# EUROPEAN PATENT SPECIFICATION

(11) **EP 2 238 129 B1**
(45) Date of publication and mention of the grant of the patent: **17.08.2011**
(21) Application number: 09705800.2
(22) Date of filing: 14.01.2009
(51) Int. Cl.: C07D 405/06

(54) **A METHOD FOR THE PREPARATION OF DARIFENACIN HYDROGEN BROMIDE**
VERFAHREN ZUR HERSTELLUNG VON DARIFENACIN-WASSERSTOFF-BROMID
PROCÉDÉ DE PRÉPARATION DE DARIFÉNACINE BROMHYDRIQUE

(30) Priority: 28.01.2008 CZ 20080045
(43) Date of publication of application: 13.10.2010
(73) Proprietor: Zentiva, K.S., 102 37 Praha 10 (CZ)
(72) Inventor: HEJTMANKOVA Ludmila, 535 01 Prelouc (CZ); JIRMAN, Josef, 100 00 Praha 10 (CZ)
(74) Representative: Jirotkova, Ivana
(86) International application number: PCT/CZ2009/000003
(87) International publication number: WO 2009/094957

(56) References cited:
- EP-A- 0 388 054
- WO-A-03/080599
- WO-A-2007/076157
- WO-A-2007/076158
- WO-A-2007/076159

## Description

### Technical Field

The invention deals with a new method of production of the hydrogen bromide of (3S)-1-[2-(2,3-dihydro-S-benzofuranyl)ethyl]-α,α-diphenyl-3-pyrrolidine acetamide, known under the non-proprietary name darifenacin and used to treat hyperactive urinary bladder and urinary incontinence.

### Background Art

The substance was first described in EP 388 054. The method of its preparation in accordance with this document is shown in the following scheme. 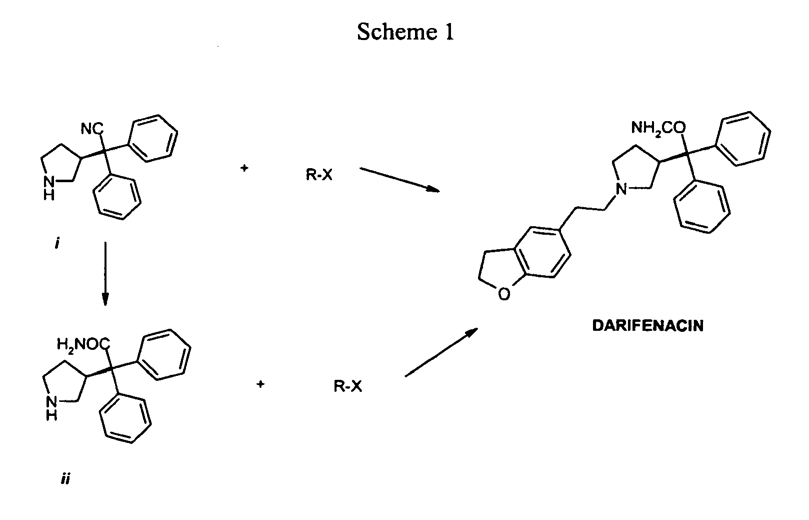 wherein the substituents R and X can be

| | R | X |
|---|---|---|
| *iii* | 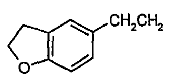 | Cl, Br, OSO₂R₁ |
| *iv* | 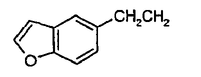 | Cl, Br, OSO₂R₁ |
| *v* | 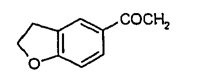 | Cl, Br |
| *vi* | 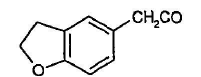 | Cl, Br |
| *vii* | 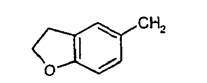 | COOH |

A particular preferable embodiment is shown in Scheme 2, wherein substance VII is alkylated with 5-(2-bromoethyl)-2,3-dihydrobenzofuran (**VIII**) in the presence of potash by reflux in acetonitrile. Crude darifenacin (**IX**) is purified using column chromatography and crystallized from diisopropylether

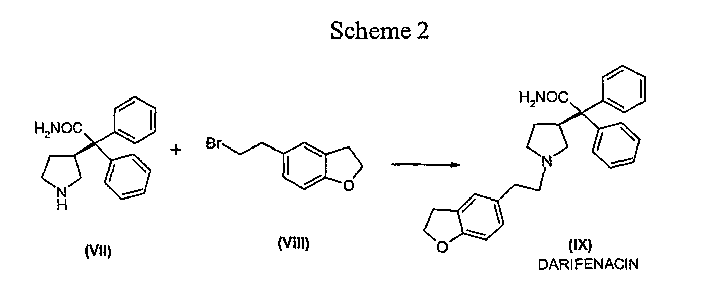

### Scheme 2: Synthesis of darifenacin by N-alkylation of pyrrolidine VII with 5-(2-bromoethyl)-2,3-dihydrobenzofuran (VIII)

Darifenacin hydrobromide is prepared by precipitation of purified darifenacin base dissolved in acetone by addition of concentrated aqueous HBr.

However, in repeated reproduction these procedures did not provide a product of an adequate quality in a reasonable industrially applicable yield.

It has been found out that a portion of the resulting darifenacin undergoes subsequent alkylation to the second stage, producing the twice substituted substance **X**. In the course of the reaction undesired reactions of 5-(2-bromoethyl)-2,3-dihydrobenzofuran **VIII** also occur, namely hydrolysis producing a hydroxy derivative (**XI**) and elimination producing a vinyl derivative (**XII**). All these reactions reduce the yield of the desired substance and complicate the preparation of high-quality API.

By reproduction of the above mentioned procedure a substance was obtained with the following contents of constituents in accordance with HPLC [%]:
VII 2.8 VIII 14.2 IX57.2 X7.8 XI1.2 XII 8.0.

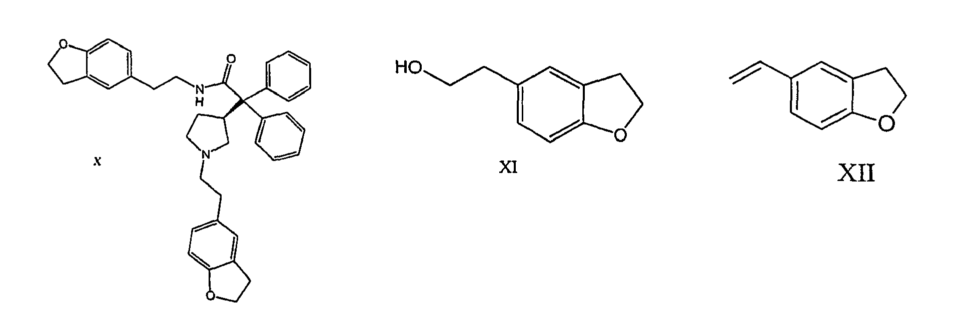

A purification procedure for darifenacin was published in WO03080599A1.

Darifenacin in t-amyl alcohol is heated with Amberlite (22 h), the solid fraction is filtered off, the solvent is evaporated from the filtrate and the residue is dissolved in toluene; a solvate of darifenacin with toluene is separated by cooling. This solvate can be directly used for the preparation of darifenacin hydrobromide (the solvate is dissolved in 2-butanol, concentrated HBr is added and the darifenacin salt is separated by cooling).

Another method of purification of darifenacin, described in the same document, is conversion of the darifenacin/toluene solvate to darifenacin hydrate (the solvate is dissolved in acetonitrile and water is added under gradual separation of darifenacin hydrate (Scheme 3)), which can be used for the preparation of salts or can be directly incorporated into pharmaceutical forms. The hydrate can be optionally converted to the hydrogen bromide in a similar way as the solvate. 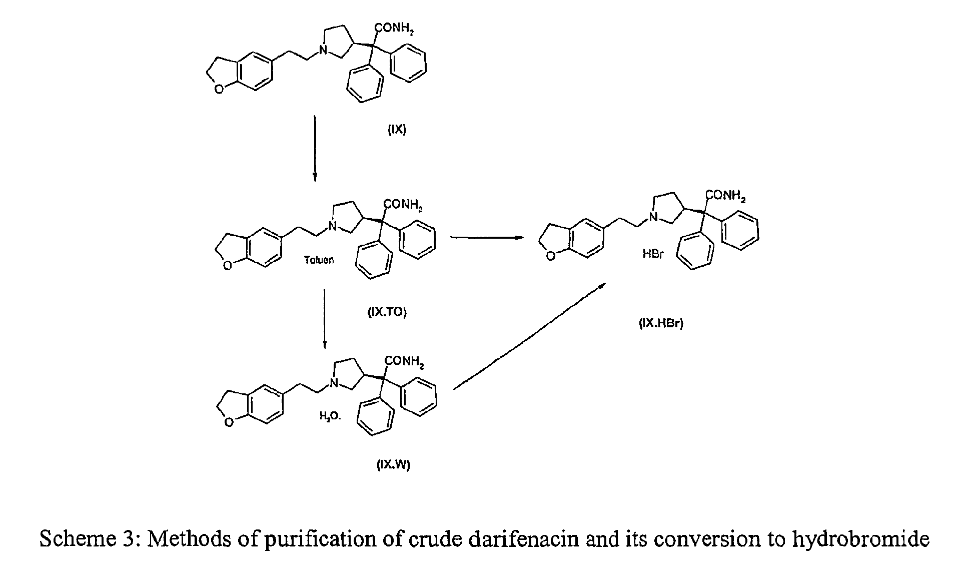 During reproduction of the purification procedure it was possible to separate a portion of substance X in the solid phase form after dissolution of crude darifenacin in toluene. However, the attempt to obtain the desired toluene solvate of darifenacin from the toluene solution was not successful during the reproduction. This means that this method does not lead to the pure substance.

WO2007076159 (TEVA) describes preparation of darifenacin from dihydrobenzofuran ethylchloride and carbamoyl(diphenylmethyl)pyrrolidine tartrate in the aqueous phase using K₂CO₃ as the base. After cooling of the reaction mixture n-butanol is added, the aqueous and organic phases are separated, acetanhydride is added and a reaction with concentrated hydrobromic acid (48%) is performed.

This method enables preparation of the substance with a satisfactory yield, ca. 77%; however, the reaction in the aqueous phase takes place in the melt, which is very thick, which causes technological problems, e.g. difficult stirring, sticking of the mixture on the walls of the reaction vessel, etc. During a reproduction of this procedure it was found that acetanhydride caused partial decomposition of the product and formation of further impurities. The crude product prepared this way cannot be converted to hydrobromide without further purification. N-butanol mentioned in the procedure is partly miscible with water, which also has a negative impact on the process yield.

Contents of constituents (HPLC [%]) in the crude product within the reproduction of the procedure in accordance with WO2007076159 (TEVA):
Reaction with dihydrobenzofuran ethylchloride:
   VII 1.9 VIII 6.1 IX 82.0 X6.3 XI not found XII not found
Reaction with dihydrobenzofuran ethylbromide:
   VII 2.8 VIII 0.5 IX 77.5 X9.5 XI2.0 XII 2.4

The above mentioned analysis of the described procedures and attempts to reproduce them have revealed that compound X is the major problem. During the application it was never possible to obtain the product that would contain less than 5% of this impurity. The substance is similar to the desired product in its character, it has similar solubility in most solvents and moreover it also changes to hydrogen bromide or other salts. For this reason it is very difficult to separate this substance by normal crystallization of the base or one of the salts of darifenacin.

While toluene has proved suitable for this function in the above-described procedures (WO03080599A1), after the separation of a portion of substance X it was not possible to obtain the desired toluene solvate of darifenacin. The procedure appears to be hardly usable without further modification and it does not lead to the desired pure product.

Processes for preparing darifenacin hydrobromide are also described in WO 2007/076157 and WO 2007/076158.

### Disclosure of Invention

The invention deals with a new method of production of (3S)-1-[2-(2,3-dihydro-5-benzofuranyl)ethyl]-α,α-diphenyl-3-pyrrolidine acetamide hydrogen bromide, known under the non-proprietary name darifenacin hydrogen bromide of formula I, 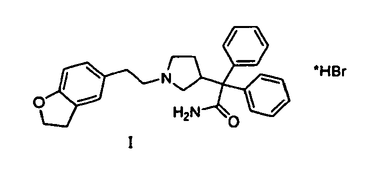 which consists in alkylating 3-(S)-(1-carbamoyl-1,1-diphenylmethyl)pyrrolidine or its salt with an organic acid, in the presence of an inorganic base, with 5-(2-bromoethyl)-2,3-dihydrobenzofuran in a heterogeneous system of the solvents water and a C6 to C9 aliphatic, alicyclic or aromatic hydrocarbon, removing the residue of water from the organic phase after separation of the two phases and adding a C3 to C9 ketone and concentrated hydrobromic acid to the mixture.

We have found out that the selectivity of the reaction is influenced by the type of the solvent used. Alkylation on pyrrolidine nitrogen is a nucleofilic substitution, which mainly proceeds by the S_{N}2 mechanism for primary alkyls. Generally, these reactions are performed in polar aprotic solvents, such as DMF, DMSO or acetonitrile (cf. EP 388 054). In protic solvents solvatation of pyrrolidine nitrogen occurs, which reduces its reactivity. In non-polar solvents the reaction does not generally have any observable speed or is very slow. These solvents are unsuitable as in the activated complex charges are generated, and thus increasing the polarity of the solvent considerably accelerates the reaction. We have surprisingly found out that when the reaction is carried out in a non-polar solvent, its speed is satisfactory, but what is especially advantageous is that the subsequent reaction producing the substance X, which principally complicates isolation of darifenacin hydrobromide in the crystalline form and substantially reduces the yield of API, is suppressed.

This method enables production of the product in a very high quality, with low contents of impurities and a relatively high yield of about 85-90 %, without the need to specially purify the crude product before its conversion to hydrobromide.

The reaction can be described by means of Scheme 4:

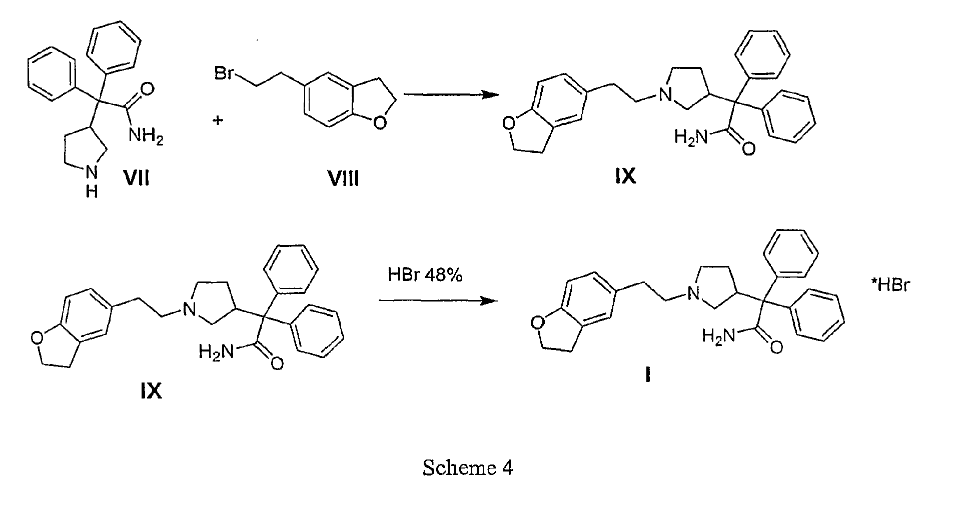

For the production of darifenacin 3-(S)-(1-carbamoyl-1,1-diphenylmethyl)pyrrolidine us used in the form of the base or as a salt selected from tartaric, oxalic, malonic, succinic or citric acid or another organic acid.

The reaction is carried out in the presence of an inorganic base selected from alkaline carbonates, hydroxides or phosphates.

The alkylation is performed in a heterogeneous system of the solvents water and a solvent selected from C6 to C9 aliphatic, alicyclic or aromatic hydrocarbons, e.g. toluene, benzene, hexane, cyclohexane, heptane, o-xylene, m-xylene or p-xylene. Cyclohexane appears to be especially suitable for this purpose.

Both the advanced intermediates react in the equimolar ratio in such a way that the reactive base of substance VII is released from the corresponding salt in an aqueous solution of an inorganic base. The base VII then passes over to the organic phase where intermediate VIII is dissolved and a reaction takes place in an aprotic environment where pyrrolidine nitrogen is not solvated and hence its reactivity is not reduced. The whole process is running one pot at a temperature of 80-110 °C. The reaction time is ca. 3.5 h. After cooling the two phases are separated and the product is isolated.

After carrying out the alkylation, separation of the aqueous and organic phases and optional extraction of the aqueous phase with the organic solvent used the darifenacin base is isolated. This isolation may proceed either by evaporation of the solvent or by crystallization of the product. A combination of both the processes is also possible, i.e. crystallization after partial evaporation of the solvent. The procedure depends on the selected solvent; e.g. in the case of toluene the solvent is distilled off and in the cases of cyclohexane the crude base is crystallized after cooling of the organic phase.

Alkylation and isolation of the darifenacin base is followed by conversion of the product - darifenacin to its hydrogen bromide. Crystalline darifenacin hydrobromide ((3S)-1-[2-(2,3-dihydro-5-benzofuranyl)ethyl]-α,α-diphenyl-3-pyrrolidine acetamide hydrogen bromide) is separated from the solution after addition of concentrated hydrobromic acid (48%) to the solution of the base in a ketone selected from dimethylketone, methylethylketone, diethylketone, dipropylketone, diisopropyll:etone, dibutylketone a ditert-butylketone or another C3-C9 ketone. Instead of ketones, one can use, with a similar result, alcohols, e.g. tert-butanol, amylalcohol, etc. For the conversion to the hydrogen bromide and the subsequent crystallization ethylmethylketone (2-butanone) appears to be the most suitable of these types of solvents.

With the procedure according to the present invention an acceptable product of a high purity can be obtained with the yield of about 85%.

In all embodiments of the invention the product was obtained with the content of substance X below 3%, in most cases below 2%. A typical composition of the product obtained in accordance with this invention can be characterized with the following content of constituents in accordance with HPLC [%]:
VII 0.5-0.7 VIII 0.9-1.2 IX89.0-90.0 X1.4-2.0 XI 0.4-0.5 XII1.1-1.5

Such base of darifenacin can be then converted without further purification into the desired salt and the substance in a purity required for API can be obtained by crystallization thereof.

The invention is further demonstrated by means of the following examples.

### Example 1

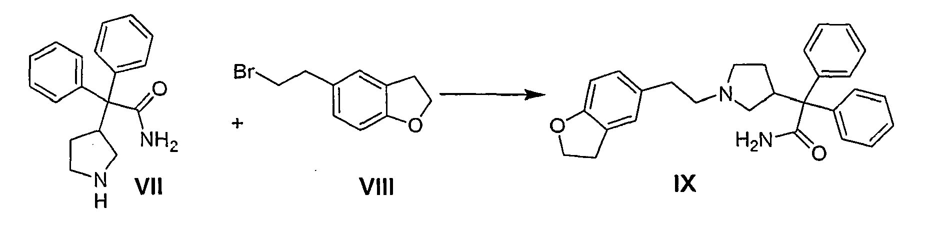

Advanced intermediate VII (4.3 g; 0.01 mol) is stirred up in an aqueous solution of potassium phosphate (9.43 g; 0.041 mol in 20 ml of water) at the laboratory temperature. A toluene solution (20 ml) of intermediate VIII (2.41 g; 0.011 mol) is added to the mixture and the mixture is heated up in an oil bath T=90 °C while being stirred for 3.5 h. After cooling the toluene layer is separated and the aqueous layer is extracted with toluene. The combined toluene extracts are shaken with water and the solvent is distilled off at a reduced pressure. The evaporation residue is dissolved in ethylmethylketone, and an equimolar amount of 48% hydrobromic acid is added. The separated darifenacin hydrobromide is filtered off and dried.
Yield: 85% of theory.

### Example 2

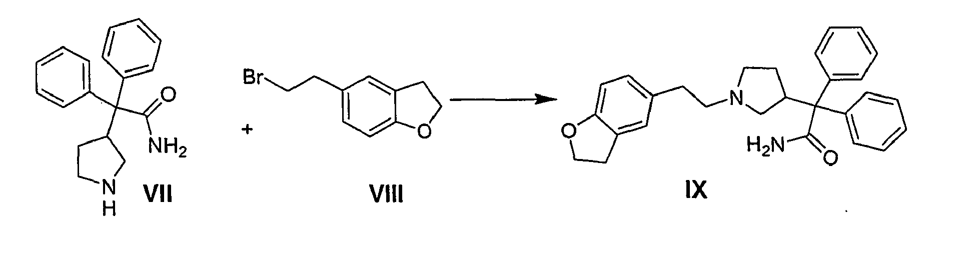

Advanced intermediate VII (4.3 g; 0.01 mol) is stirred up in an aqueous solution of potassium carbonate (6.1 g; 0.044 mol in 20 ml of water) at the laboratory temperature. A toluene solution (20 ml) of intermediate VIII (2.41 g; 0.011 mol) is added to the mixture and the mixture is heated in an oil bath T=90 °C while being stirred for 3.5 h. After cooling the toluene layer is separated and the aqueous layer is extracted with toluene. The combined toluene extracts are shaken with water and the solvent is distilled off at a reduced pressure. The evaporation residue is dissolved in ethylmethylketone, and an equimolar amount of 48% hydrobromic acid is added. The separated darifenacine hydrobromide is filtered off and dried.
Yield: 86% of theory.

### Example 3

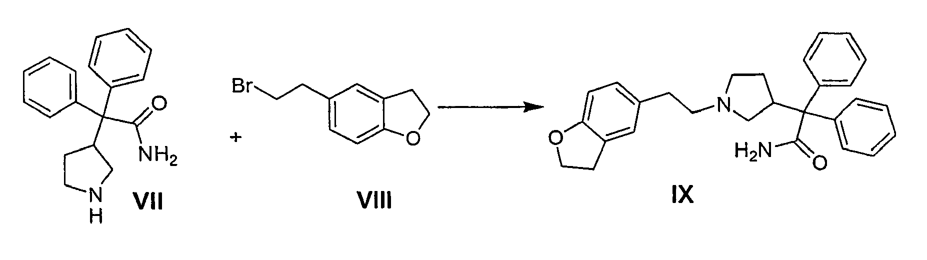

Advanced intermediate VII (4.3 g; 0.01 mol) is stirred up in an aqueous solution of potassium phosphate (9.43 g; 0.041 mol in 20 ml of water) at the laboratory temperature. A solution of intermediate VIII (2.41 g; 0.011 mol) in cyclohexane (20 ml) is added to the mixture and the mixture is heated in an oil bath T=90 °C while being stirred for 3.5 h. The layers are separated while hot. The cyclohexane solution is cooled to the laboratory temperature under intensive stirring. This way the darifenacin base is separated. The product is filtered off and dried. The base is dissolved in ethylmethylketone, and an equimolar amount of 48% hydrobromic acid is added. The separated darifenacin hydrobromide is filtered off and dried.
Yield: 85% of theory.

## Claims

1. A method for the preparation of (3S)-1-[2-(2,3-dihydro-5-benzofuranyl)ethyl]-α,α-diphenyl-3-pyrrolidine acetamide hydrogen bromide, ***characterized in* that** 3-(S)-(1-carbamoyl-1,1-diphenylmethyl)pyrrolidine or its salt with an organic acid is alkylated in the presence of an inorganic base with 5-(2-bromoethyl)-2,3-dihydrobenzofuran in a heterogeneous system of the solvents water and an organic solvent selected from C6 to C9 aliphatic, alicyclic or aromatic hydrocarbons, after separation of the two phases the crude darifenacin base is isolated, which is converted to the hydrobromide by addition of a C3 to C9 ketone or a C3 to C9 alcohol and concentrated hydrobromic acid.

2. The method according to claim 1, **characterized in that** 3-(S)-(1-carbamoyl-1,1-diphenylmethyl)pyrrolidine is used in the base form.

3. The method according to claim 1, **characterized in that** 3-(S)-(1-carbamoyl-1,1-diphenylmethyl)pyrrolidine is used in the form of a salt selected from salts with tartaric, oxalic, malonic, succinic or citric acid.

4. The method according to claims 1 or 3, **characterized in that** 3-(S)-(1-carbamoyl-1,1-diphenylmethyl)pyrrolidine is used in the form of a salt with tartaric acid.

5. The method according to any of the preceding claims, **characterized in that** the inorganic base is selected from alkali carbonates, hydroxides or phosphates.

6. The method according to claim 5, **characterized in that** the base is potassium phosphate or its hydrate.

7. The method according to any of the preceding claims, **characterized in that** the heterogeneous system of solvents is water and an organic solvent selected from toluene, benzene, hexane, cyclohexane, heptane, o-xylene, m-xylene and p-xylene.

8. The method according to claim 7, **characterized in that** the organic solvent is cyclohexane.

9. The method according to any of the preceding claims, **characterized in that** the solvent for the conversion of (3S)-1-[2-(2,3-dihydro-5-benzofuranyl)ethyl]α,α-diphenyl-3-pyrrolidine acetamide base to its hydrogen bromide is selected from dimethylketone, methylethylketone, diethylketone, dipropylketone, diisopropylketone, dibutylketone and ditert-butylketone.

10. The method according to claim 9, **characterized in that** the solvent is ethylmethylketone.

## Patentansprüche

1. Verfahren zur Erstellung von (3S)-1-[2-(2,3-Dihydro-5-benzofuranyl)ethyl]-α,α-diphenyl-3-pyrrolidinacetamid-Hydrobromid, **dadurch gekennzeichnet, dass** 3-(S)-(1-Carbamoyl-1,1-diphenylmethyl)pyrrolidin oder dessen Salz mit einer organischen Säure in Gegenwart einer anorganischen Base mit 5-(2-Bromoethyl)-2,3-dihydrobenzofuran in einem heterogenen System der Lösungsmittel Wasser und eines organischen Lösungsmittels, das aus C6 bis C9 aliphatischen, alicyclischen oder aromatischen Kohlenwasserstoffen ausgewählt ist, alkyliert wird, nach der Trennung der zwei Phasen die rohe Darifenacinbase isoliert wird, die durch Hinzufügen eines C3 bis C9 Ketons oder eines C3 bis C9 Alkohols und konzentrierter Bromwasserstoffsäure in das Hydrobromid umgewandelt wird.

2. Verfahren gemäß Anspruch 1, **dadurch gekennzeichnet, dass** 3-(S)-(1-Carbamoyl-1,1-diphenylmethyl)pyrrolidin in Form der Base verwendet wird.

3. Verfahren gemäß Anspruch 1, **dadurch gekennzeichnet, dass** 3-(S)-(1-Carbamoyl-1,1-diphenylmethyl)pyrrolidin in Form eines Salzes verwendet wird, das aus Salzen mit Weinsäure, Oxalsäure, Malonsäure, Bernsteinsäure oder Zitronensäure ausgewählt ist.

4. Verfahren gemäß Anspruch 1 oder 3, **dadurch gekennzeichnet, dass** 3-(S)-(1-Carbamoyl-1,1-diphenylmethyl)pyrrolidin in Form eines Salzes mit Weinsäure verwendet wird.

5. Verfahren gemäß mindestens einem vorhergehenden Anspruch, **dadurch gekennzeichnet**, das die anorganische Base aus Alkalicarbonaten, Hydroxiden oder Phosphaten ausgewählt ist.

6. Verfahren gemäß Anspruch 5, **dadurch gekennzeichnet, dass** die Base Kaliumphosphat oder dessen Hydrat ist.

7. Verfahren gemäß mindestens einem vorhergehenden Anspruch, **dadurch gekennzeichnet, dass** das heterogene Lösungsmittelsystem Wasser und ein organisches Lösungsmittel ist, das aus Toluol, Benzol, Hexan, Cyclohexan, Heptan, o-Xylol, m-Xylol und p-Xylol ausgewählt ist.

8. Verfahren gemäß Anspruch 7, **dadurch gekennzeichnet, dass** das organische Lösungsmittel Cyclohexan ist.

9. Verfahren gemäß mindestens einem vorhergehenden Anspruch, **dadurch gekennzeichnet, dass** das Lösungsmittel zur Umwandlung der (3S)-1-[2-(2,3-Dihydro-5-benzofuranyl)ethyl]-α,α-diphenyl-3-pyrrolidinacetamid-Base in ihr Hydrobromid aus Dimethylketon, Methylethylketon, Diethylketon, Dipropylketon, Diisopropylketon, Dibutylketon und Di-tert-butylketon ausgewählt ist.

10. Verfahren gemäß Anspruch 9, **dadurch gekennzeichnet, dass** das Lösungsmittel Ethylmethylketon ist.

## Revendications

1. Un procédé de préparation de l'acide bromhydrique de (3S)-1-[2-(2,3-dihydro-5-benzofuranyl)éthyl]-α,α-diphényl-3-pyrrolidine acétamide, **caractérisé en ce que** la 3-(S)-(1-carbamoyl-1,1-diphénylméthyl)pyrrolidine ou l'un de ses sels avec un acide organique est alkylé en présence d'une base inorganique avec du 5-(2-bromoéthyl)-2,3-dihydrobenzofurane dans un système hétérogène de solvant à base d'eau et de solvant organique choisi parmi les hydrocarbones aliphatique, alicyclique ou aromatique en C6 à C9, et **en ce que** après séparation des deux phases, la base darifénacine brute est isolée, puis est convertie en le bromhydrate par addition d'une cétone en C3 à C9 ou d'un alcool en C3 à C9 et d'acide bromhydrique concentré.

2. Le procédé selon la revendication 1, **caractérisé en ce que** la 3-(S)-(1-carbamoyl-1,1-diphénylméthyl)pyrrolidine est utilisée sous forme de base.

3. Le procédé selon la revendication 1, **caractérisé en ce que** la 3-(S)-(1-carbamoyl-1,1-diphénylméthyl)pyrrolidine est utilisée sous la forme d'un sel choisi parmi les sels d'acide tartrique, oxalique, malonique, succinique ou citrique.

4. Le procédé selon la revendication 1 ou 3, **caractérisé en ce que** la 3-(S)-(1-carbamoyl-1,1-diphénylméthyl)pyrrolidine est utilisée sous la forme d'un sel d'acide tartrique.

5. Le procédé selon l'une quelconque des revendications précédentes, **caractérisé en ce que** la base inorganique est choisie parmi les carbonates, hydroxydes ou phosphates alcalins.

6. Le procédé selon la revendication 5, **caractérisé en ce que** la base est du phosphate de potassium ou son hydrate.

7. Le procédé selon l'une quelconque des revendications précédentes, **caractérisé en ce que** le système hétérogène de solvants est à base d'eau et d'un solvant organique choisi parmi le toluène, le benzène, l'hexane, le cyclohexane, l'heptane, les o-xylène, m-xylène et p-xylène.

8. Le procédé selon la revendication 7, **caractérisé en ce que** le solvant organique est le cyclohexane.

9. Le procédé selon l'une quelconque des revendications précédentes, **caractérisé en ce que** le solvant utilisé pour la conversion de la base 3(S)-1-[2-(2,3-dihydro-5-benzofuranyl)éthyl]-α,α-diphényl-3-pyrrolidine acétamide en son bromhydrate est choisi parmi la diméthylcétone, la méthyléthylcétone, la diéthylcétone, la dipropylcétone, la diisopropylcétone, la dibutylcétone et la di-tert-butylcétone.

10. Le procédé selon la revendication 9, **caractérisé en ce que** le solvant est l'éthylméthylcétone.
